# NEUE EUROPÄISCHE PATENTSCHRIFT

(11) **EP 1 590 065 B2**
(45) Veröffentlichungstag und Bekanntmachung des Hinweises auf die Entscheidung über den Einspruch: **29.10.2014**
(45) Hinweis auf die Patenterteilung: 13.04.2011
(21) Anmeldenummer: 04704209.8
(22) Anmeldetag: 22.01.2004
(51) Int. Cl.: B01D 39/16, A61M 1/36

(54) **FILTER ZUR ABTRENNUNG VON LEUKOZYTEN AUS VOLLBLUT/ODER BLUTPRÄPARATEN, VERFAHREN ZUR HERSTELLUNG DIESER FILTER, VORRICHTUNG UND VERWENDUNG**
FILTER FOR THE SEPARATION OF LEUKOCYTES FROM WHOLE BLOOD OR BLOOD PREPARATIONS, METHOD FOR PRODUCTION OF SAID FILTER, CORRESPONDING DEVICE AND USE THEREOF
FILTRES POUR SEPARER DES LEUCOCYTES PRESENTS DANS DU SANG TOTAL/OU DANS DES PREPARATIONS SANGUINES, PROCEDE DE PRODUCTION DE CES FILTRES, DISPOSITIF ASSOCIE ET UTILISATION ASSOCIEE

(30) Priorität: 24.01.2003 IT TO20030039
(43) Veröffentlichungstag der Anmeldung: 02.11.2005
(73) Patentinhaber: Fresenius Hemocare Italia S.r.l., I-41032 Cavezzo, (Modena) (IT)
(72) Erfinder: BONAGUIDI, Paolo, I-56100 Pisa (IT); MARI, Giorgio, I-41037 Mirandola (IT); VERRI, Paolo, I-41037 Mirandola (IT)
(74) Vertreter: Brandt, Maximilian
(86) Internationale Anmeldenummer: PCT/EP2004/000489
(87) Internationale Veröffentlichungsnummer: WO 2004/064980

(56) Entgegenhaltungen:
- EP-A- 0 554 460
- EP-A- 1 238 694

## Beschreibung

Die vorliegende Erfindung betrifft Filter zur Abtrennung von Leukozyten aus Vollblut und/oder Blutpräparaten, ein Verfahren zur Herstellung dieser Filter sowie Vorrichtungen, die diese Filter enthalten. Des weiteren bezieht sich die vorliegende Erfindung auf die Verwendung dieser Filter zur Abtrennung von Leukozyten aus Vollblut und/oder Blutpräparaten.

Zur Behandlung von Patienten mit bestimmten Krankheiten finden Konzentrate von Blutkomponenten Verwendung, die den Patienten zugeführt werden. Die Blutkomponentenkonzentrate können gewonnen werden, in dem Vollblut einer Dichtezentrifugation unterworfen wird. Sie sollten eine möglichst geringe Konzentration von Leukozyten aufweisen, da diese Immunreaktionen beim Patienten auslösen können. Aus diesem Grund wird versucht, Leukozyten aus Blutpräparaten zu entfernen.

Filter zur Abtrennung von Leukozyten aus Blutpräparaten sind allgemein bekannt. So beschreiben beilspielsweise die Druckschriften US 4,830,548, US 5,445,736 und US 5,580,465 Filter, die aus Konzentraten von Blutplättchen (PLT) Leukozyten (WBC) abtrennen können. Derartige Filter eignen sich jedoch nicht zur Abtrennung von Leukozyten aus Vollblut, da die Erythrozyten (RC) bei Berührung mit dem Filtermaterial verändert werden.

Die Abtrennung von Leukozyten aus Vollblut wird in US 5,895,575 beschrieben. Das Filtermaterial umfasst ein hydrophobes Trägermaterial, beispielsweise ein Faservlies aus Polyethylenterephthalat, auf das ein hydrophiles, in Wasser lösliches Polysaccharid aufgetragen wird. Problematisch hierbei ist die relativ geringe Abtrennung der Leukozyten aus Vollblut. Im Vergleich zu den in US 4,880,548 beschriebenen Filtern trennen die mit den Polysacchariden beschichteten Filter wesentlich weniger Leukozyten aus dem Blutpräparat ab. Zwar lässt sich diese Abtrennung mit Hilfe von Nitrocellulosefiltern verbessern. Derartige Systeme sind jedoch aufwendig und teuer. Darüber hinaus ist die Wasserlöslichkeit des Beschichtungsmaterials problematisch. Da Vollblut grosse Mengen an Wasser enthält, kann das Beschichtungsmaterial ausgewaschen werden. Hierdurch werden jedoch Thrombozyten oder Platulozyten auf dem Filtermaterial zurückgehalten. Problematisch ist hierbei insbesondere, dass dieser Verlust kontinuierlich stattfindet. Zwar wird in US 5,895,575 eine Vernetzungsreaktion beschrieben, die die Löslichkeit herabsetzen soll. Eine derartige Vernetzung führt jedoch zu weiteren Problemen. So können insbesondere Rückstände des Vernetzungsmittels in den Blutkreislauf gelangen. Des weiteren kann eine sehr starke Vernetzung zu einer verminderten Durchlässigkeit des Filters führen. Eine zu geringe Vernetzung hingegen kann ein Auswaschen nur unvollständig verhindern.

Dokument EP0554460 offenbart eine Vorrichtung mit einem Filter zur Abtrennung von Leukozyten aus Vollblut aufweisend ein aus Polyester Faservlies, bei dem eine Modifikation der Filteroberfläche durch Beschichtung mit hydrophilen Polymeren ausgeführt wird.

Eine Aufgabe der vorliegenden Erfindung liegt darin, Filter zur Abtrennung von Leukozyten aus Vollblut und/oder Blutpräparaten zu schaffen, die zwar Leukozyten besonders gut zurückhalten, Blutplättchen und Erythrozyten aber möglichst ungehindert passieren lassen.

Darüber hinaus besteht eine Aufgabe der vorliegenden Erfindung darin, Filter bereitzustellen, die eine besonders hohe Haltbarkeit aufweisen. Insbesondere sollten die Filter ihre Durchlässigkeit für Blutplättchen auch nach längerem Gebrauch behalten.

Eine weitere Aufgabe der vorliegenden Erfindung ist, Filter zur Verfügung zu stellen, die einfach und kostengünstig herstellbar sind. Insbesondere sollten die eingesetzten Materialien problemlos gereinigt werden können, um den medizinischen Anforderungen zu genügen.

Gelöst werden diese Aufgaben sowie weitere, die zwar nicht wörtlich genannt werden, sich aber aus den hierin diskutierten Zusammenhängen ableiten lassen oder aus diesen zwangsläufig ergeben, mit den Merkmalen des Anspruchs 1. Zweckmässige Abwandlungen der erfindungsgemässen Filter sind Gegenstand der auf Anspruch 1 rückbezogenen Unteransprüche.

Die unabhängigen Ansprüche 17, 20 und 22 beziehen sich auf das Verfahren zur Herstellung der Filter und eine Vorrichtung zur Abtrennung von Leukozyten aus Vollblut sowie die Verwendung der Filter.

Dadurch, dass die Oberfläche von Filtern, die ein hydrophobes Trägermaterial sowie ein polymeres Beschichtungsmaterial aufweisen, eine kritische Oberflächenbenetzungsspannung im Bereich von 50 bis 80 dyn/cm aufweisen, wobei das polymere Beschichtungsmaterial durch eine Polyreaktion von Mischungen erhältlich ist, die hydrophobe und hydrophile Monomere umfassen, gelingt es Filter zur Abtrennung von Leukozyten aus Vollblut zur Verfügung zu stellen, die ein besonders hohes Rückhaltevermögen gegenüber Leukozyten aufweisen.

Durch die erfindungsgemässen Massnahmen werden insbesondere folgende Vorteile erzielt:
⇒ Die erfindungsgemässen Filter weisen eine hohe Durchlässigkeit für Blutplättchen und Erythrozyten auf.
⇒ Die Filter der vorliegenden Erfindung zeigen eine hohe Haltbarkeit, wobei insbesondere die Durchlässigkeit für Blutplättchen bei längerem Gebrauch sehr hoch bleibt. Hierbei zeigen die erfindungsgemäßen Filter eine sehr geringe Auswaschung.
⇒ Die Filter können kostengünstig hergestellt werden, wobei die verwendeten Materialien auf einfache Weise gereinigt werden können, um so den medizinischen Anforderungen zu genügen.
⇒ Die Filter zeigen eine hervorragende Leistungsfähigkeit. So zeichnen sich die erfindungsgemäßen Filter durch eine hohe Abtrennung von Leukozyten bei besonders hoher Durchlässigkeit und geringem Rückhaltevermögen gegenüber Blutplättchen aus. Des Weiteren können die erfindungsgemäßen Filter bei relativ hohen Flussraten betrieben werden. Darüber hinaus wird von den Filtermaterialien relativ wenig Material benötigt, um eine bestimmte Filterleistung zu erzielen.

Die Filter der vorliegenden Erfindung weisen ein hydrophobes Trägermaterial auf. Derartige Materialien sind dem Fachmann bekannt. Hierzu gehören insbesondere flächige Gebilde, wie beispielsweise Membranen und Faservliese, die Teilchen aufgrund von Grössenunterschieden trennen können. Der Begriff hydrophob kennzeichnet, dass das Trägermaterial in Wasser nicht löslich ist, wobei die Löslichkeit in Wasser bei 20°C kleiner oder gleich 0,1 g/l ist.

Die Membranen können unter anderem aus Polycarbonaten, Acrylpolymeren, Polyvinylchloriden, Polyamiden und Nitrocellulose gefertigt werden. Die Porengrösse dieser Membranen liegt vorzugsweise im Bereich von 2 bis 20 µm, ohne dass hierdurch eine Beschränkung erfolgen soll.

Besonders geeignete Faservliese sind unter anderem aus Polyester, Cellulose, Polyamiden und Polypropylenen erhältlich. Von diesen Materialien sind insbesondere Polyester bevorzugt, wie beispielsweise Polyethylenterephthalat (PET), Polybutylenterephthalat (PBT) und Polytrimethylenterephthalat (PTT). Derartige Vliese werden bevorzugt aus Fasern hergestellt, die einen Durchmesser im Bereich von 0,5 bis 8 µm, insbesondere 1 bis 4 µm, besonders bevorzugt 1,8 bis 3 µm aufweisen. Hierbei sind insbesondere kristalline Fasern bevorzugt, wobei die Kristallinität mindestens 50%, insbesondere mindestens 80% beträgt. Diese Grösse kann durch Röntgenbeugung gemessen werden.

Die Oberflächendichte der Faservliese liegt im Allgemeinen im Bereich von 10 bis 200 g/m², insbesondere im Bereich von 15 bis 100 g/m², besonders bevorzugt von 20 bis 60 g/m² ohne dass hierdurch eine Beschränkung erfolgen soll. Die Oberflächendichte kann beispielsweise gemäß ISO 9073-1:1989 und EDANA ERT 40.9-90 bestimmt werden. Die Dicke des Vlieses kann in einem weiten Bereich liegen. Vorzugsweise beträgt die Dicke des Vlieses vor der Beschichtung ca. 0,1 bis 1,3 mm, vorzugsweise 0,3 bis 0,7 und besonders bevorzugt 0,4 bis 0,6 mm, wobei diese Werte gemäß ISO 9073-1:1997-2 gemessen werden können. Hierbei beträgt die gemessene Oberfläche 25 cm² bei einem Druck von 0,5 kPa. Die Dichte von bevorzugten Faservliesen liegt im Bereich von 0,05 bis 0,50 g/cm³, insbesondere 0,15 bis 0,30 g/cm³.

Des weiteren umfasst ein erfindungsgemässer Filter ein polymeres Beschichtungsmaterial, das auf das Trägermaterial aufgebracht wird.

Der Filter zeigt nach der Beschichtung des hydrophoben Trägermaterials mit dem polymeren Beschichtungsmittel eine kritische Oberflächenbenetzungsspannung (critical wetting surface tension) im Bereich von 50 bis 80 dyn/cm, insbesondere 55 bis 72 dyn/cm, vorzugsweise 55 bis 69 dyn/cm. Diese Grösse kann gemäss dem in den US-Patenten US5,445,736 und 4,880,548 beschriebenen Verfahren bestimmt werden.

Um eine derartige Oberflächenbenetzungsspannung zu erzielen, umfassen die polymeren Beschichtungsmittel hydrophile Gruppen, wobei Gruppen bevorzugt sind, die bei pH 7,0 in Wasser ionisch geladen sind. Die hydrophilen Monomere gemäß der Erfindung sind ausgewählt aus der Gruppe bestehend aus Vinylpyrrolidon und Acrylsäure.

Dementsprechend können die polymeren Beschichtungsmittel beispielsweise durch Polyreaktionen erhalten werden, bei denen hydrophile und hydrophobe Monomere eingesetzt werden. Die Begriffe hydrophob und hydrophil sind in der Fachwelt allgemein bekannt und beziehen sich auf die Benetzbarkeit der aus den Monomeren erhaltenen Polymere mit Wasser. Die Benetzbarkeit mit Wasser wird durch polare Gruppen, die im Polymer nach der Polyreaktion vorhanden sind, erhöht. Dementsprechend sind Monomere hydrophil, die polare Gruppen, beispielsweise Hydroxyl-, Ether-, Carboxyl-. Carboxylat-, Amid-, Amin-, Pyrrolidin-, Pyrrolidon-, Lactam-, Sulfonat-, Phosphonat- oder Guanidin-Gruppen, und einen relativ geringen Anteil an unpolaren Gruppen, insbesondere Kohlenwasserstoffgruppen, enthalten. Die Hydrophilie vieler Monomere kann beispielsweise über die Löslichkeit eines Homopolymerisats bestimmt werden. Falls zur Herstellung der Polymere unterschiedliche Monomere notwendig sind, beispielsweise bei vielen Polyestern, so bestimmt sich die Hydrophilie des Monomers über die Löslichkeit eines Polymers, das neben dem Monomer ein passendes zweites Monomer mit einer Ethylengruppe umfasst. Zur Bestimmung der Hydrophilie von Terephthalsäure wird diese beispielsweise mit Glykol umgesetzt. Die Hydrophilie ergibt sich aus der Löslichkeit von PET.

Im Allgemeinen ist ein Monomer hydrophil, falls die Löslichkeit des Homopolymerisats oder eines zuvor dargelegten Polymerisats größer oder gleich 1 g/l, insbesondere 5 g/l und besonders bevorzugt 10 g/l beträgt.

Im Allgemeinen ist ein Monomer hydrophob, falls die Löslichkeit des Homopolymerisats oder eines zuvor dargelegten Polymerisats kleiner 1 g/l, insbesondere kleiner oder gleich 0,5 g/l und besonders bevorzugt kleiner oder gleich 0,1 g/l beträgt.

Dem Fachmann ist bekannt, dass die Löslichkeit der Polymere vom Molekulargewicht abhängig ist. Die zuvor dargelegten Löslichkeitswerte gelten für ein über das Gewicht gemittelte Molekulargewicht von 200 000 g/mol, wobei dieses Molekulargewicht über dem Fachmann bekannte Methoden, beispielsweise die Gel-Permeations-Chromatographie bestimmt werden kann. Die Temperatur zur Bestimmung dieser Löslichkeit liegt bei 20°C.

Die zuvor dargelegten Polyreaktionen sind allgemein bekannt. Diese umfassen insbesondere radikalische Polymerisationen, Polyadditionen und Polykondensationen.

Die Hydrophobie von radikalisch polymerisierbaren Monomeren kann über die Mischbarkeit bzw. Löslichkeit dieser Monomere in Wasser abgeschätzt werden. Hydrophobe Monomere zeichnen sich im Allgemeinen durch eine sehr begrenzte Mischbarkeit mit Wasser aus, wohingegen hydrophile Monomere mit Wasser gut mischbar sind.

Gemäß einem besonderen Aspekt der vorliegenden Erfindung zeichnen sich bevorzugte hydrophobe, radikalisch polymerisierbare Monomere durch eine Löslichkeit in Wasser von kleiner oder gleich 20 g/l, insbesondere 16 g/l und besonders bevorzugt 10 g/l bei 20°C aus. Im Vergleich hierzu beträgt die Löslichkeit von bevorzugten hydrophilen, radikalisch polymerisierbaren Monomere bei 20°C mindestens 50 g/l, vorzugsweise mindestens 100 g/l und besonders bevorzugt sind hydrophile Monomere in jedem Verhältnis mit Wasser bei 20°C mischbar.

Zu den radikalisch polymerisierbaren, hydrophoben Monomeren zählen unter anderem Vinylester, wie Vinylacetat; Alkene, wie Ethylen, Propylen, Hexen-1, Hepten-1, Vinylcyclohexan, 3,3-Dimethyl-1-propen, 3-Methyl-1-diisobutylen, 4-Methylpenten-1; und Alkyl(meth)acrylate, die sich von gesättigten Alkoholen ableiten, wie beispielsweise Methylacrylat, Ethyl(meth)acrylat, Propyl(meth)acrylat, n-Butyl(meth)acrylat, tert.-Butyl(meth)acrylat, wobei der Ausdruck (Meth)acrylate Methacrylate und Acrylate sowie Mischungen aus beiden umfasst.

Die hydrophilen Monomere sind ausgewählt aus der Gruppe bestehend aus Vinylpyrrolidon und Acrylsäure.

Besonders bevorzugte Monomere weisen bei pH 7,0 mindestens eine ionische Gruppe auf. Hierzu gehören unter anderem die zuvor genannten Monomere, die mindestens eine basische oder eine saure Gruppe aufweisen.

Zu den hydrophoben Monomeren, die in einer Polyadditions- oder Polykondensationsreaktion eingesetzt werden können, zählen unter anderem Alkenoxide, wie Butylenoxid, Hexylenoxid; Monomere die zur Herstellung von Polyestern, Polycarbonaten und Polyurethanen verwendet werden, wie Isophorondiisocyanat, Hexamethylendiisocyanat, Hexan-1,6-diol und Terephthalsäure.

Die zuvor genannten hydrophilen und hydrophoben Monomere können einzeln oder als Mischung von zwei oder mehreren eingesetzt werden, um statistische Copolymere, Blockcopolymere und/oder Pfropfcopolymere zu erhalten. Von diesen Polymeren sind statistische Copolymere besonders bevorzugt, wobei diese Polymere einen, über die Polymerkette betrachtet, im Wesentlichen zufälligen Einbau der verschiedenen Monomere aufweisen.

Gemäss einem besonderen Aspekt der vorliegenden Erfindung umfassen die Mischungen zur Herstellung des polymeren Beschichtungsmaterials 1 bis 40 Gew.-%, bevorzugt 4 bis 25 Gew.-% hydrophile Monomere und 99 bis 60 Gew.-%, insbesondere 96 bis 75 Gew.-% hydrophobe Monomere, ohne dass hierdurch eine Beschränkung erfolgen soll.

Die Polyreaktionen zur Herstellung der polymeren Beschichtungsmittel sind allgemein bekannt und beispielsweise in Ullmann's Encyclopedia of Industrial Chemistry, Fifth Edition on CDROM mit weiteren Literaturhinweisen beschrieben.

Des weiteren können aber auch hydrophobe Polymere so umgesetzt werden, dass diese die zuvor dargelegten Gruppen aufweisen. Dies kann beispielsweise durch bekannte Pfropfreaktionen erfolgen, wobei auf hydrophobe Polymere hydrophile Monomere aufgepfropft werden. Darüber hinaus können auch hydrophobe Polymere, wie Polymethyl(meth)acrylate, partiell versteift werden, um Polymere zu erhalten, die Carboxygruppen aufweisen.

Das Molekulargewicht der Polymere, die im polymeren Beschichtungsmaterial enthalten sind, kann in weiten Bereichen schwanken. Im Allgemeinen liegt das Gewichtsmittel des Molekulargewichts M_{w} im Bereich von 10 000 bis 200 000 g/mol, vorzugsweise 20 000 bis 100 000 g/mol.

Die-Polymere des Beschichtungsmaterials weisen vorzugsweise einen Polydispersitätsindex M_{w}/Mₙ im Bereich von 1 bis 5, besonders bevorzugt 1,2 bis 4 auf, ohne dass hierdurch eine Beschränkung erfolgen soll.

Das Zahlenmittel des Molekulargewichts Mₙ und das Gewichtsmittel des Molekulargewichts können durch Gel-Permeations-Chromatographie (GPC) bestimmt werden.

Das polymere Beschichtungsmaterial weist eine Löslichkeit in Wasser bei 20°C kleiner oder gleich 1 g/l, vorzugsweise kleiner oder gleich 0,5 g/l auf. Die geringe Löslichkeit des Polymers bezieht sich insbesondere auch auf den Zustand des Polymers bevor dies auf das Trägermaterial aufgebracht wird. Diese Grösse kann gravimetrisch bestimmt werden.

Die Polymere des Beschichtungsmaterials können auf einfache Weise durch Ausfällen aus Wasser von Restmonomeren und anderen Nebenprodukten gereinigt werden. Hierzu wird eine Lösung der Polymeren einfach in eine grosse Menge Wasser gegeben, wodurch die in Wasser löslichen Bestandteile entfernt werden können. Zu den Lösungsmitteln, in denen das Polymer gelöst werden kann, gehören unter anderem Alkohole, insbesondere Ethanol; Ketone, insbesondere Aceton; und Alkane, insbesondere Hexan.

Nach dem Ausfällen können die Polymere getrocknet und nochmals in organischem Lösungsmittel aufgenommen werden.

Gemäß einem besonderen Aspekt der vorliegenden Erfindung kann nur ein sehr geringer Teil des beschichteten Trägermaterials durch Wasser bei 37°C extrahiert werden. Bei einer Flussrate von 1 l/h können innerhalb von 1 Stunde höchstens 0,5 Gew.-%, vorzugsweise höchstens 0,3 Gew.-%, bezogen auf das Gesamtgewicht des beschichteten Filters, extrahiert werden. Dieser Anteil kann gravimetrisch bestimmt werden.

Die geringe Extrahierbarkeit bevorzugter beschichteter Filtermaterialien kann sich unter anderem in einem niedrigen Oxidationslevel widerspiegeln. Zur Bestimmung des Oxidationslevels kann ein Filter mit 30 Filterlagen bei einer Oberflächendichte von ca. 60 g/m² und einer Filterfläche von ca. 50 cm² mit 250 ml Wasser 2 Stunden bei 37°C extrahiert werden. Anschließend können 20 ml der extrahierten Lösung mit 20 ml einer 0,01 N Kaliumpermanganatlösung in Gegenwart von 1 ml Schwefelsäure behandelt werden, wobei die Reaktionsmischung 3 Minuten unter Rückfluss erhitzt wird. Anschließend kann ein Kaliumjodid-Überschuss zugegeben werden. Der Anteil an oxidierbaren Verbindungen im Extrakt bestimmt sich über eine Titration des gebildeten Jods mit einer 0,01 N Natriumthiosulfatlösung. Über eine Blindprobe ergibt sich der Oxidationslevel als die Volumendifferenz von Extrakt zu Wasser. Bevorzugte beschichtete Filtermaterialien zeichnen sich durch einen Oxidationslevel ΔOx kleiner oder gleich 3 ml, insbesondere kleiner oder gleich 2 ml aus.

Die Oberflächendichte der beschichteten Filter ist im Allgemeinen etwas höher als die Oberflächendichte der Faservliese. Im Allgemeinen liegt dieser Wert im Bereich von 16 bis 240 g/m², insbesondere im Bereich von 20 bis 110 g/m², besonders bevorzugt im Bereich von 26 bis 70 g/m² ohne dass hierdurch eine Beschränkung erfolgen soll.

Das Trägermaterial sowie das polymere Beschichtungsmaterial sind vorzugsweise biokompatibel, wobei hierunter die hohe Verträglichkeit dieser Materialien zu verstehen ist.

Im Allgemeinen bedeckt das polymere Beschichtungsmittel mindestens 50%, vorzugsweise mindestens 90%, besonders bevorzugt mindestens 99% der Oberfläche des Trägermaterials. Diese Grösse kann, je nach Kombination Trägermaterial- Beschichtungsmittel über verschiedene in der Fachwelt bekannte Methoden ermittelt werden, wobei bei relativen Methoden üblich Eichkurven angefertigt werden. Zu den Methoden gehören unter anderem spektroskopische Verfahren, wie die ESCA -Verfahren, sowie optische Verfahren, bei denen beispielsweise das Beschichtungsmaterial durch Bildung von Komplexen mit Jod gefärbt wird, wohingegen das Trägermaterial durch Jod nicht gefärbt wird. Über den Anteil der durch Jod verfärbten Oberfläche, kann die Bedeckung der Oberfläche bestimmt werden.

Die Menge an Beschichtungsmaterial, die auf das Trägermaterial aufgetragen wird, richtet sich sowohl nach den Eigenschaften des Trägermaterials als auch nach denen des Beschichtungsmaterials, wobei eine kritische Oberflächenbenetzungsspannung im Bereich von 50 bis 80 dyn/cm erzielt werden muss. Dementsprechend kann diese Grösse in einem weiten Bereich liegen. Im Allgemeinen umfasst der Filter 1 bis 25 Gew.-% polymeres Beschichtungsmaterial und 99 bis 75 Gew.-% Trägermaterial.

Gemäss einem besonderen Aspekt der vorliegenden Erfindung kann das polymere Beschichtungsmaterial aus dem Filter mit einem der organischen Lösungsmittel extrahiert werden. Die Extraktion kann insbesondere bei Temperaturen leicht unter dem Siedepunkt des jeweiligen Lösungsmittels erfolgen.

Die erfindungsgemässen Filter zur Abtrennung von Leukozyten aus Vollblut können auf einfache Weise hergestellt werden, indem man ein hydrophobes Trägermaterial mit einer Lösung beschichtet, die ein polymeres Beschichtungsmaterial und ein Lösungsmittel umfasst, und anschliessend das Lösungsmittel aus dem mit einem polymeren Beschichtungsmittel versehenen Trägermaterial entfernt.

Die Beschichtung des Trägermaterials kann auf jede bekannte Weise erfolgen. Beispielhaft seien Tauchverfahren genannt.

Der Anteil des Lösungsmittels der Lösung richtet sich nach der auf das Trägermaterial aufzubringenden Menge an polymerem Beschichtungsmaterial. Bevorzugt umfassen die Lösungen 50 bis 99,5 Gew.-%, besonders bevorzugt 70 bis 99 Gew.-% Lösungsmittel.

Die Filter der vorliegenden Erfindung werden im Allgemeinen in Vorrichtungen zur Reinigung von Blutpräparaten verwendet. Derartige Vorrichtungen umfassen üblich einen Behälter, beispielsweise einen Beutel, in dem sich das zu reinigende Blut befindet, ein von dem Filter in zwei Kammern unterteiltes Gehäuse sowie einen weiteren Behälter, in dem das Filtrat aufgefangen wird, wobei diese Teile der Vorrichtung durch Schlauchleitungen verbunden sind. Der Transport des Blutes vom ersten Behälter über den Filter zum zweiten Behälter kann beispielsweise allein durch Schwerkraft oder auch durch eine Pumpe, bevorzugt eine Peristaltikpumpe erfolgen. Derartige Vorrichtungen sind beispielhaft in US 5,445,736 und US 5,162,102 beschrieben.

Der in dem Gehäuse befindliche Filter kann ein oder mehrere Filterlagen umfassen. Gemäss einem besonderen Aspekt umfassen die Filter 15 bis 50, bevorzugt 20 bis 40 Filterlagen.

Nachfolgend wird die Erfindung durch Beispiele und Vergleichsbeispiele eingehender erläutert, ohne dass die Erfindung auf diese Beispiele beschränkt werden soll.

### Beispiel 1

Ein statistisches Copolymer aus Vinylacetat und Vinylpyrrolidon wurde durch radikalische Polymerisation hergestellt, wobei ein Gewichtsverhältnis Vinylacetat zu Vinylpyrrolidon von 7 eingesetzt wurde. Hierzu wurden die flüssigen Reagenzien durch Destillation unter Stickstoffatmosphäre gereinigt. 98,5g Vinylacetat, 14g Vinylpyrrolidon und 0,59g Azobisisobutyronitril wurden in 200 ml Aceton gegeben. Die erhaltene Mischung wurde unter Stickstoffatmosphäre für 72 Stunden bei ca. 54°C gerührt. Anschließend wurde das erhaltene Polymer mit 200 ml Hexan extrahiert. Aus der Hexanlösung wurde das Polymer durch Zugabe von 1000 ml Wasser gefällt. Das isolierte Polymer wurde in Aceton gelöst. Es wurde ein statistisches Copolymer erhalten, dessen Gewichtsmittel des Molekulargewichts gemäss Gel-Permeations-Chromatographie 56 611 g/mol und dessen Polydispersitätsindex 3,5 betrug, wobei als stationäre Phase ein Styrol-Divinylbenzol-Polymer und als fluide Phase Chloroform verwendet wurde.

Das so erhaltene Polymer wurde mehrfach durch Ausfällen in Wasser gereinigt, um Monomerreste zu entfernen. Hierzu wurde das Polymer in Aceton gelöst und bei Raumtemperatur in Wasser gegeben. Der Niederschlag wurde getrocknet und wiederum in Aceton gelöst.

Anschliessend wurde eine acetonische Lösung des Polymers hergestellt, die 4 Gew.-% Polymer enthielt. In diese Lösung wurde ein Polybutylenterephthalat-Vlies getaucht, das eine Oberflächendichte von 50 g/m² aufwies und aus Fasern mit einem Durchmesser von 2 µm hergestellt wurde (erhältlich von Fresenius HemoCare Italia).

Nach dem Tauchen wurde überschüssige Lösung abgetropft. Anschließend wurde das beschichtete Vlies getrocknet, wobei eine übliche Vorrichtung eingesetzt wurde (Behandlungsgeschwindigkeit 5 mt/min bei 95°C). Die Oberflächendichte betrug 58,8 g/m².

In ein rechteckiges Filtergehäuse mit einer Filterfläche von 50 cm² wurden 30 Lagen des beschichteten Vlieses eingebaut. Durch den so erhaltenen Filter wurden ca. 500 ml Vollblut mittels Gravitationskraft filtriert. Die Blutplättchenkonzentrationen vor und nach der Filtration wurden mit einem automatischen Analysiergerät (Coulter Gen-s; Beckman) bestimmt. Die Anzahl der Leukozyten wurden mit einem Hemocytometer von Nageotte gemessen, wobei diese Zahl nach der Filtration in einer Burker-Kammer unter Verwendung einer verdünnten Lösung (Leukoplate Lösung von Sobioda) gezählt wurden.

Die erhaltenen Ergebnisse sind in Tabelle 1 dargelegt.

Des weiteren wurde der so erhaltene Filter auf extrahierbare Verbindungen untersucht. Hierzu wurden 250 ml destilliertes Wasser 2 Stunden durch den Filter mit einer Peristaltikpumpe in einem Kreislauf gepumpt, wobei die Temperatur 37°C und die Flussrate von 11/h betrugen. Die so erhaltene Mischung wurde auf Rückstände untersucht, wobei 0,1 Gew.-% Rückstände festgestellt wurden, bezogen auf das Gewicht des Filters vor der Extraktionsbehandlung.

Des weiteren wurde das erhaltene Extrakt auf oxidierbare Substanzen untersucht. Hierzu wurden 20 ml der extrahierten Lösung mit 20 ml einer 0,01 N Kaliumpermanganatlösung in Gegenwart von 1 ml Schwefelsäure behandelt, wobei die Reaktionsmischung 3 Minuten unter Rückfluss erhitzt wurde. Anschließend wurde ein Überschuss an Kaliumjodid bei 20°C zugegeben. Das hierdurch gebildete Jod wurde mit einer 0,01 N Natriumthiosulfatlösung titriert. Über eine Blindprobe ergibt sich der Oxidationslevel als die Volumendifferenz von Extrakt zu Wasser. Der Oxidationslevel betrug 0,4 ml.

### Beispiel 2

Das Beispiel 1 wurde im Wesentlichen wiederholt, wobei jedoch anstatt eines Gewichtsverhältnisses Vinylacetat zu Vinylpyrrolidon von 7 ein Gewichtsverhältnis von 12 eingesetzt wurde.

Es wurde ein statistisches Copolymer erhalten, dessen Gewichtsmittel des Molekulargewichts gemäss Gel-Permeations-Chromatographie 56 611 g/mol und dessen Polydispersitätsindex 3,5 betrug.

Die Oberflächendichte des beschichteten Vlieses betrug 60,2 g/m².

Die Ergebnisse der Vollblutfiltration sind ebenfalls in Tabelle 1 dargelegt.

Die Analyse auf extrahierbare Verbindungen ergab einen Oxidationslevel von 1,2 ml, wobei 0,3 Gew.-% Rückstände im Extrakt vorhanden waren, bezogen auf das Gewicht des Filters vor der Behandlung.

### Beispiel 3

Das Beispiel 1 wurde im Wesentlichen wiederholt, wobei jedoch anstatt eines Gewichtsverhältnisses Vinylacetat zu Vinylpyrrolidon von 7 ein Gewichtsverhältnis von 4 eingesetzt wurde. Die Oberflächendichte des beschichteten Vlieses betrug 59,9 g/m².

Die Ergebnisse der Vollblutfiltration sind ebenfalls in Tabelle 1 dargelegt.

### Vergleichsbeispiel 1

Das Beispiel 1 wurde im Wesentlichen wiederholt, wobei jedoch unbeschichtetes Vlies aus Polybutylenterephthalat zur Filtration von Vollblut verwendet. Die Ergebnisse sind ebenfalls in Tabelle 1 dargelegt.

**Tabelle 1**

| | Wiederfindungsrate von Blutplättchen [%] | Anzahl der Leukozyten in 500 ml Filtrat* [10⁶] |
|---|---|---|
| Beispiel 1 | 80 ± 2 | 0,8 ± 0,2 VA/VP 7P |
| Beispiel 2 | 70 ± 5 | 0,5 ± 0,2 VA/VP 12P |
| Beispiel 3 | 78 ± 9 | 0,6 ± 0,4 VA/VP 4P |
| Vergleichsbeispiel 1 | 0 | 0,1 ± 0,1 unbeschichtet |

| | | |
|---|---|---|
| *Die Zahl vor der Filtration betrug ca. 3*10⁹ | | |

## Patentansprüche

1. Filter zur Abtrennung von Leukozyten aus Vollblut und/oder Blutpräparaten aufweisend ein hydrophobes Trägermaterial sowie ein polymeres Beschichtungsmaterial, wobei die Oberfläche des Filters eine kritische Oberflächenbenetzungsspannung im Bereich von 50 bis 80 dyn/cm aufweist, **dadurch gekennzeichnet, dass** das polymere Beschichtungsmaterial durch eine Polyreaktion von Mischungen erhältlich ist, die hydrophobe und hydrophile Monomere umfassen,
und **dadurch gekennzeichnet, dass** die hydrophilen Monomere ausgewählt sind aus der Gruppe bestehend aus Vinylpyrrolidon, Acrylsäure.

2. Filter nach Anspruch 1, **dadurch gekennzeichnet, dass** höchstens 0,5 Gew.-% des Filters bei 37 °C durch Wasser extrahiert werden können.

3. Filter nach Anspruch 2, **dadurch gekennzeichnet, dass** die Mischungen 1 bis 40 Gew. -% hydrophile Monomere und 99 bis 60 Gew.-% hydrophobe Monomere umfassen.

4. Filter nach einem der Ansprüche 1 bis 3, **dadurch gekennzeichnet, dass** die hydrophoben Monomere ausgewählt sind aus der Gruppe bestehend aus Vinylacetat, Methylmethacrylat, Isophorondiisocyanat.

5. Filter nach einem der Ansprüche 1 bis 4, **dadurch gekennzeichnet, dass** das Beschichtungsmittel bei pH 7,0 ionische Gruppen aufweist.

6. Filter nach einem der Ansprüche 1 bis 5, **dadurch gekennzeichnet, dass** das Beschichtungsmittel mindestens 90% der Oberfläche des Trägermaterials bedeckt.

7. Filter nach einem der Ansprüche 1 bis 6, **dadurch gekennzeichnet, dass** die Polyreaktion zur Herstellung des Beschichtungsmaterial eine radikalische Polymerisation ist.

8. Filter nach einem der Ansprüche 1 bis 7, **dadurch gekennzeichnet, dass** das polymere Beschichtungsmaterial ein Gewichtsmittel des Molekulargewichts M_{w} im Bereich von 10 000 bis 200 000 g/mol aufweist.

9. Filter nach einem der Ansprüche 1 bis 8, **dadurch gekennzeichnet, dass** das polymere Beschichtungsmaterial einen Polydispersitätsindex M_{w}/Mₙ im Bereich von 1 bis 5 aufweist.

10. Filter nach einem der Ansprüche 1 bis 9, **dadurch gekennzeichnet, dass** das polymere Beschichtungsmaterial ein statistisches Copolymer ist (im Fall von Polyadditionen nicht statistisch).

11. Filter nach einem der Ansprüche 1 bis 10, **dadurch gekennzeichnet, dass** das Trägermaterial kristalline Fasern umfasst.

12. Filter nach Anspruch 11, **dadurch gekennzeichnet**, das die Fasern Polyester enthalten.

13. Filter nach Anspruch 11 oder 12, **dadurch gekennzeichnet, dass** die Fasern einen Durchmesser im Bereich von 0,5 bis 8 µm aufweisen.

14. Filter nach einem der Ansprüche 11 bis 13, **dadurch gekennzeichnet, dass** das Trägermaterial ein Faservlies ist.

15. Filter nach einem der Ansprüche 1 bis 14, **dadurch gekennzeichnet, dass** der Filter 1 bis 25 Gew. -% polymeres Beschichtungsmaterial umfasst.

16. Filter nach einem der Ansprüche 1 bis 15, **dadurch gekennzeichnet, dass** die Oberflächendichte des Filters im Bereich von 20 bis 110 g/m² liegt.

17. Verfahren zur Herstellung von Filtern nach einem der Ansprüche 1 bis 16, **dadurch gekennzeichnet, dass** man ein hydrophobes Trägermaterial mit einer Lösung beschichtet, die ein polymeres Beschichtungsmaterial und ein Lösungsmittel umfasst, und anschließend das Lösungsmittel aus dem mit einem polymeren Beschichtungsmittel versehenen Trägermaterial entfernt.

18. Verfahren nach Anspruch 17, **dadurch gekennzeichnet, dass** man das hydrophobe Trägermaterial in eine Lösung taucht.

19. Verfahren nach Anspruch 17 oder 18, **dadurch gekennzeichnet, dass** man das polymere Beschichtungsmittel durch Ausfällen in Wasser reinigt.

20. Vorrichtung zur Abtrennung von Leukozyten aus Vollblut und/oder Blutpräparaten umfassend mindestens einen Filter nach einem der Ansprüche 1 bis 16.

21. Vorrichtung nach Anspruch 20, **dadurch gekennzeichnet, dass** der Filter 15 bis 50 Filterlagen umfasst.

22. Verwendung eines Filters nach einem der Ansprüche 1 bis 16 zur Abtrennung von Leukozyten aus Vollblut.

## Claims

1. A filter for the separation of leukocytes from whole blood and/or blood preparations, comprising a hydrophobic support material and a polymeric coating material, wherein the surface of the filter exhibits a critical surface wetting tension in the range of 50 to 80 dyn/cm, **characterised in that** the polymeric coating material is obtainable by a polyreaction of mixtures which comprise hydrophobic and hydrophilic monomers, and **characterised in that** the hydrophilic monomers are selected from the group consisting of vinyl pyrrolidone, acrylic acid.

2. The filter according to claim 1, **characterised in that** a maximum of 0.5% by weight of the filter can be extracted by water at 37 ° C.

3. The filter according to claim 2, **characterised in that** the mixtures comprise 1 to 40% by weight of hydrophilic monomers and 99 to 60 % by weight of hydrophobic monomers.

4. The filter according to any of claims 1 to 3, **characterised in that** the hydrophobic monomers are selected from the group consisting of vinyl acetate, methyl methacrylate, isophorone diisocyanate.

5. The filter according to any of claims 1 to 4, **characterised in that** the coating agent exhibits ionic groups at a pH of 7.0.

6. The filter according to any of claims 1 to 5, **characterised in that** the coating agent covers at least 90% of the surface of the support material.

7. The filter according to any of claims 1 to 6, **characterised in that** the polyreaction for producing the coating material is a radical polymerisation reaction.

8. The filter according to any of claims 1 to 7, **characterised in that** the polymeric coating material exhibits a weight average of the molecular weight M_{w} ranging from 10,000 to 200,000 g/mol.

9. The filter according to any of claims 1 to 8, **characterised in that** the polymeric coating material exhibits a polydispersity index M_{w}/Mₙ ranging from 1 to 5.

10. The filter according to any of claims 1 to 9, **characterised in that** the polymeric coating material is a statistical copolymer (non statistical in the case of polyaddition reactions).

11. The filter according to any of claims 1 to 10, **characterised in that** the support material incorporates crystalline fibres.

12. The filter according to claim 11, **characterised in that** the fibres comprise polyester.

13. The filter according to claim 11 or 12, **characterised in that** the fibres exhibit a diameter ranging from 0.5 to 8 ,.m.

14. The filter according to any of claims 11 to 13, **characterised in that** the support material is a fibre non-woven fabric.

15. The filter according to any of claims 1 to 14, **characterised in that** the filter comprises 1 to 25% by weight of polymeric coating material.

16. The filter according to any of claims 1 to 15, **characterised in that** the surface density of the filter ranges from 20 to 110 g/m².

17. A method for producing filters according to any of claims 1 to 16, **characterised in that** a hydrophobic support material is coated with a solution comprising a polymeric coating material and a solvent, and **in that** the solvent is then removed from the support material provided with a polymeric coating agent.

18. The method according to claim 17, **characterised in that** the hydrophobic support material is immersed in a solution.

19. The method according to claim 17 or 18, **characterised in that** the polymeric coating material is cleaned by precipitation in water.

20. A device for separating leukocytes from whole blood and/or blood preparations comprising at least one filter according to any of claims 1 to 16.

21. The device according to claim 20, **characterised in that** the filter comprises 15 to 50 filter layers.

22. Use of a filter according to any of claims 1 to 16 for separating leukocytes from whole blood.

## Revendications

1. Filtre pour séparer des leucocytes de sang total et/ou de préparations sanguines présentant un matériau substrat hydrophobe, et un matériau de revêtement polymère, **caractérisé en ce que** le matériau de revêtement polymère peut être obtenu par une polyréaction de mélanges qui comprennent des monomères hydrophobes et hydrophiles, la surface du filtre présentant une tension superficielle de mouillage critique dans une plage de 50 à 80 dyn/cm, et **caractérisé en ce que** les monomères hydrophiles sont choisis dans le groupe comprenant la vinylpyrrolidone, l'acide acrylique.

2. Filtre selon la revendication 1, **caractérisé en ce que**, au plus, 0,5 % en poids du filtre peuvent être extraits par l'eau à 37°C.

3. Filtre selon la revendication 2, **caractérisé en ce que** les mélanges comprennent 1 à 40 % en poids de monomères hydrophiles et 99 à 60 % en poids de monomères hydrophobes.

4. Filtre selon l'une des revendications 1 à 3, **caractérisé en ce que** les monomères hydrophobes sont choisis dans le groupe comprenant l'acétate de vinyle, le méthylméthacrylate, le diisocyanate d'isophorone.

5. Filtre selon l'une des revendications 1 à 4, **caractérisé en ce que** l'agent de revêtement présente des groupes ioniques à pH 7,0..

6. Filtre selon l'une des revendications 1 à 5, **caractérisé en ce que** l'agent de revêtement couvre au moins 90 % de la surface du matériau substrat.

7. Filtre selon les revendications 1 à 6, **caractérisé en ce que** la polyréaction pour la production du matériau de revêtement est une polymérisation radicalaire.

8. Filtre selon l'une des revendications 1 à 7, **caractérisé en ce que** le matériau de revêtement polymère présente une moyenne en poids du poids moléculaire M_{w} de l'ordre de 10 000 à 200 000 g/mole.

9. Filtre selon l'une des revendications 1 à 8, **caractérisé en ce que** le matériau de revêtement polymère présente un indice de polydispersité M_{w}/Mₙ de l'ordre de 1 à 5.

10. Filtre selon l'une des revendications 1 à 9, **caractérisé en ce que** le matériau de revêtement polymère est un copolymère statistique (dans le cas de polyadditions, un copolymère non statistique).

11. Filtre selon l'une des revendications 1 à 10, **caractérisé en ce que** le matériau substrat comprend des fibres cristallines.

12. Filtre selon la revendication 11, **caractérisé en ce que** les fibres contiennent du polyester.

13. Filtre selon la revendication 11 ou 12, **caractérisé en ce que** les fibres présentent un diamètre de l'ordre de 0,5 à 8 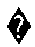 m.

14. Filtre selon l'une des revendications 11 à 13, **caractérisé en ce que** le matériau substrat est un tissu non tissé.

15. Filtre selon l'une des revendications 1 à 14, **caractérisé en ce que** le filtre comprend 1 à 25 % en poids de matériau de revêtement polymère.

16. Filtre selon l'une des revendications 1 à 15, **caractérisé en ce que** la densité superficielle du filtre est de l'ordre de 20 à 110 g/m².

17. Procédé de production de filtres selon l'une des revendications 1 à 16, **caractérisé en ce que** l'on recouvre un matériau substrat hydrophobe avec une solution qui comprend un matériau de revêtement polymère et un solvant, et ensuite, le solvant est éliminé du matériau substrat comportant un agent de revêtement polymère.

18. Procédé selon la revendication 17, **caractérisé en ce que** l'on plonge le matériau substrat hydrophobe dans une solution.

19. Procédé selon la revendication 17 ou 18, **caractérisé en ce que** l'on purifie l'agent de revêtement polymère par précipitation dans l'eau.

20. Dispositif de séparation de leucocytes du sang total et/ou de préparations sanguines comprenant au moins un filtre selon l'une des revendications 1 à 16.

21. Dispositif selon la revendication 20, **caractérisé en ce que** le filtre comprend 15 à 50 couches filtrantes.

22. Utilisation d'un filtre selon l'une des revendications 1 à 16, pour séparer des leucocytes du sang total.
